# EUROPÄISCHE PATENTANMELDUNG

(11) **EP 4 699 577 A2**
(43) Veröffentlichungstag der Anmeldung: **25.02.2026**
(21) Anmeldenummer: 26150357.7
(22) Anmeldetag: 13.11.2019
(51) Int. Cl.: A61F 2/42

(54) **ZIRKONIUMBESCHICHTETE IMPLANTATKOMPONENTE UND DEREN VERWENDUNG**

(62) Teilanmeldung aus: 19208824.3
(71) Anmelder: Waldemar Link GmbH & Co. KG, 22339 Hamburg (DE)
(72) Erfinder: CSASZAR, Richard, 23795 Bad Segeberg (DE); LINK, Helmut D., 22397 Hamburg (DE)
(74) Vertreter: AWA Denmark A/S

(57) **Zusammenfassung**

Die vorliegende Offenbarung betrifft eine Implantatkomponente mit zumindest einem Verbindungsabschnitt, wobei der Verbindungsabschnitt zumindest abschnittsweise mit einer Zr-Beschichtung beschichtet ist und die Beschichtung eine Dicke von 1-20 µm, bevorzugt 1-6 µm aufweist. Ferner betrifft die vorliegende Offenbarung eine modulare Endoprothese mit einer Implantatkomponente, die Verwendung einer Zr-Beschichtung zur Vorbeugung gegen Spaltkorrosion und/oder Reibkorrosion (fretting corrosion), sowie die Verwendung einer Implantatkomponente bei Patienten mit einer Metallallergie.

## Beschreibung

### TECHNISCHES GEBIET

Die vorliegende Offenbarung betrifft eine Implantatkomponente mit einem Verbindungsabschnitt zum Verbinden mit einer weiteren Implantatkomponente, sowie ein Implantat mit zumindest einer Implantatkomponente. Die vorliegende Offenbarung betrifft ferner die Verwendung einer Implantatkomponente bzw. eines Implantats.

### STAND DER TECHNIK

Endoprothesen, beispielsweise Hüftendoprothesen können einen modularen Aufbau aufweisen, d. h. sie sind aus zumindest zwei getrennt voneinander wählbaren Komponenten zusammengesetzt. Dieser Aufbau verleiht den Prothesen eine hohe Anpassungsfähigkeit an die individuellen Anforderungen eines Patienten.

Die individuellen Anforderungen eines Patienten können beispielsweise konkrete Abmaße, Geometrien, Werkstoffkombinationen und/oder Befestigungsmechanismen beinhalten. Die Verwendung modularer Prothesen erlaubt es, die Vielzahl an Anforderungen zu berücksichtigen, ohne dass Implantate für jede mögliche Permutation vorrätig gehalten oder individuell hergestellt werden müssen.

Einzelne Implantatkomponenten einer modular aufgebauten Endoprothese können über Verbindungsabschnitte zu einer Endoprothese bzw. einem Implantat zusammengesetzt werden. Hierbei haben sich vor allem Verjüngungsverbindungen bewährt. Eine Verjüngungsverbindung kann beispielsweise einen im Wesentlichen kegelförmig ausgebildeten Vorsprung (männliche Verjüngung) und eine Öffnung mit einem sich verjüngenden Querschnitt (weibliche Verjüngung) aufweisen. Die männliche Verjüngung und die weibliche Verjüngung weisen jeweils eine Verjüngungsachse auf, die während des Einführens des Vorsprungs in die Öffnung (d.h., während des Verbindens der Implantatkomponenten) zusammenfallen. Die Verjüngungsverbindung ist somit auch selbstzentrierend.

Wenn die männliche Verjüngung in die weibliche Verjüngung eingebracht ist, und die Implantatkomponenten in Richtung der Verjüngungsachse mit einer Kraft beaufschlagt werden, führt dies zu einer bezüglich der Verjüngungsachse radial orientierten elastischen Aufweitung der weiblichen Verjüngung und zu einer Kompression der männlichen Verjüngung. Die aus Aufweitung und Kompression resultierenden Rückfederkräfte wiederum erzeugen eine Klemmkraft, die bei Selbsthemmung zu einer reibschlüssigen Verbindung der Implantatkomponenten führt.

Solche Verjüngungsverbindungen werden beispielsweise bei Hüftendoprothesen vorgesehen. Einfach modulare Hüftendoprothesen weisen eine derartige Verbindung beispielsweise zwischen einem Prothesenschaft und einem Hüftkopf (Gelenkkopf) auf. Ferner sind mehrfach modulare Hüftendoprothesen bekannt, die ein Zwischenstück aufweisen, das zwischen dem Prothesenschaft und dem Hüftkopf angeordnet ist, um eine bessere Anpassung der Endoprothese an die Anatomie des Patienten zu ermöglichen. In diesem Fall können beispielsweise der Prothesenschaft und das Zwischenstück und/oder das Zwischenstück und der Hüftkopf mit einer zuvor beschriebenen Verjüngungsverbindung verbunden werden.

Es hat sich jedoch gezeigt, dass bei den oben beschriebenen modularen Endoprothesen Spaltkorrosion und/oder Reibkorrosion in der Verjüngungsverbindung auftreten kann (Fachbegriff: fretting bzw. fretting corrosion). Insbesondere betrifft diese Erscheinung die Kontaktflächen der Implantatkomponenten, also die Mantelfläche der männlichen Verjüngung und die innere Umfangsfläche der weiblichen Verjüngung.

Derartige Korrosionserscheinungen können mitunter zu einem fatalen Versagen des Implantats führen. Dieses Versagen tritt insbesondere als Abbrechen der männlichen Verjüngung auf. Als Ursache für die Korrosion werden mitunter Mikrobewegungen zwischen den Verbindungsflächen zweier Implantatkomponenten sowie die daraus resultierenden Spannungen verantwortlich gemacht. Die Korrosion kann sich mitunter in Form von Rissen und/oder Abrieb einer Passivschicht auf der Oberfläche des Implantatmaterials äußern. Dabei wird angemerkt, dass Korrosionserscheinungen nicht nur bei Metall-Metall-Paarungen auftreten können, sondern auch bei Metall-Keramik-Paarungen, wie beispielsweise bei Hüftendoprothesen mit Metallschaft und Keramikkopf, und insbesondere bei Paarungen mit verschiedenen Metallen (z.B. Schaft aus Titanium und Kopf aus CoCrMo)
Weiterhin wird davon ausgegangen, dass derartige Verjüngungsverbindungen zwar nach dem oben beschriebenen Prinzip funktionieren, die Verbindung jedoch fertigungsbedingt nicht über die gesamte Länge der Verjüngung stattfindet. So wurde festgestellt, dass die Verjüngungsverbindung vor allem im proximalen Bereich, d. h. am Ende der Verjüngung, zustande kommt. Die Folge sind höhere Spannungen an dessen Oberfläche, welche die oben beschriebenen Korrosionserscheinungen begünstigen. Verstärkt wird dieser Effekt zudem dadurch, dass Verjüngungsverbindungen im Regelfall spanend, insbesondere durch Drehen, gefertigt werden. Dabei entsteht eine hierfür charakteristische wellenförmige Oberfläche im Mikrometerbereich. Auch dies kann beabsichtigte lokale Verformungen und Spannungsspitzen bei einer Verjüngungsverbindung hervorrufen.

Neben der obigen Materialversagensverformung führen die vorstehend beschriebenen Korrosionserscheinungen zudem regelmäßig zur Freisetzung von Metallionen, Metalloxiden, Metallorganophosphaten und/oder von kleinen Metallpartikeln, die wiederum die mechanischen Abriebserscheinungen verstärken. Dadurch können bei Patienten Schmerzen, eine aseptische Lockerung der Endoprothese und/oder negative Folgewirkungen für das umliegende Gewebe auftreten. So kann insbesondere eine Metallose entstehen, d. h. ein unnatürliches Vorkommen von Abriebpartikeln im Gewebe. Eine mögliche Folge hiervon ist das Entstehen sogenannter Pseudotumore. Auch sind allergischen Reaktionen möglich, die ebenfalls eine Revision der Prothese erforderlich machen können.

Um den vorstehend beschriebenen Problemen entgegenzuwirken, wurden bisher mitunter konstruktive und fertigungstechnische Maßnahmen ergriffen. Beispielsweise wurde angestrebt, dass Maß der Korrosionserscheinungen durch präzisiere Herstellungsverfahren oder durch angepasste Kontaktflächengeometrien zu reduzieren. Aus der DE 102014206151 A1 ist zudem eine Verjüngungsverbindung mit einer TiNb-Beschichtung bekannt, die gegenüber unbeschichteten Verbindungen eine geringere Korrosionsanfälligkeit aufweist.

Als weitere Möglichkeit wurde eine Verwendung von Nitridbeschichtungen in Erwägung gezogen, um die Abriebeigenschaften von Implantatkomponenten zu verbessern. Diese werden zum Beispiel für die Laufflächen von Endoprothesen verwendet. Jedoch vorherrschen in einem Verbindungsabschnitt hier andere Parameter. Aufgrund der obigen beschriebenen Materialverformung ist eine weiche verformbare Beschichtung erforderlich.

Da also in Verbindungsabschnitten mit oder ohne Beschichtung weder ein frühzeitiges Versagen noch Wechselwirkungen mit Abriebmaterial ausgeschlossen werden können, besteht weiterhin das Bestreben, die Korrosionserscheinungen über das bisher erreichte Maß hinaus weiter zu reduzieren.

### ZUSAMMENFASSUNG DER ERFINDUNG

Dem Gegenstand der vorliegenden Offenbarung lag die Aufgabe zugrunde, den vorstehend beschriebenen Problemen entgegenzuwirken und Verbesserungen bei einer Implantatkomponente mit einem Verbindungsabschnitt und insbesondere Verbesserungen bezüglich Spaltkorrosion und/oder Reibkorrosion (fretting corrosion) zu erwirken. Diese Aufgabe wird durch eine Implantatkomponente nach Anspruch 1, eine modulare Endoprothese nach Anspruch 10, eine Verwendung nach Anspruch 13 oder eine Verwendung nach Anspruch 14 gelöst. Bevorzugte Ausführungsformen sind in den Unteransprüchen angegeben.

Eine Implantatkomponente gemäß der vorliegenden Offenbarung weist zumindest einen Verbindungsabschnitt auf, der zumindest abschnittsweise mit einer elementaren Zr-Beschichtung beschichtet ist. Bevorzugt ist der gesamte Verbindungsabschnitt mit einer elementaren Zr-Beschichtung beschichtet. Besonders bevorzugt sind zumindest diejenigen Flächen des Verbindungsabschnitts, die dazu ausgebildet sind, mit Flächen eines Verbindungsabschnittsgegenstücks einer anderen Implantatkomponente in Kontakt zu treten, mit einer elementaren Zr-Beschichtung beschichtet.

Die elementare Zr-Beschichtung weist eine Dicke zwischen 1 µm und 20 µm, bevorzugt zwischen 1 µm und 6 µm auf. Bei Zr handelt es sich im Rahmen der vorliegenden Offenbarung um elementares Zirconium (Element mit der Ordnungszahl 40 im Periodensystem), nicht jedoch um Zirkon (Zr[SiO₄]) oder andere zirconiumbasierte Minerale oder Keramiken. Zirconiumbasierte Oxide, die sich an der Oberfläche einer Zr-Beschichtung in Form einer Passivschicht ausbilden können, werden einer Zr-Beschichtung gemäß der vorliegenden Offenbarung zugeordnet.

Die vorstehend beschriebene Implantatkomponente kann mit einer gegenüber dem Stand der Technik signifikant verringerten Anfälligkeit für Spaltkorrosion und/oder Reibkorrosion im Bereich des Verbindungsabschnitts in Verbindung gebracht werden. Diese Eigenschaft ist im Wesentlichen auf die Wahl des Beschichtungsmaterials (Zr) in Kombination mit der Schichtdicke zurückzuführen.

Mit dem weiter unten beschriebenen Messverfahren wurde gezeigt, dass ein Verbindungsabschnitt mit einer elementaren Zr-Beschichtung (mit einer offenbarungsgemäßen Schichtdicke) eine signifikant geringere Anfälligkeit für Spaltkorrosion und/oder Reibkorrosion (fretting corrosion) aufweist als beispielsweise ein Verbindungsabschnitt mit Titan-Niob (TiNb) oder ein Verbindungsabschnitt, der mit einer Kobaltbasislegierung(z.B. CoCrMo) ausgebildet ist.

Durch die geringere Anfälligkeit für Spaltkorrosion und/oder Reibkorrosion (fretting corrosion) wird angenommen, dass die Passivschicht an der Oberfläche der Zr-Beschichtung wesentlich stabiler ist als die Passivschicht anderer metallischer Beschichtungen oder Werkstoffe. Zudem sprechen bisherige Untersuchungen für ein hohes Maß an Biokompatibilität, was insbesondere gegen allergische Reaktionen vorbeugt.

Weiterhin wird davon ausgegangen, dass die geringe Anfälligkeit der Zr-Beschichtung für Korrosion auf die mechanischen Eigenschaften dieser Beschichtung zurückzuführen ist. Insbesondere besitzt die Beschichtung eine ausreichende Duktilität. Als Folge hiervon wird ein gleichmäßigerer Kontakt beim Verbindungsabschnitt ermöglicht und somit Spannungsspitzen abgebaut. Dies ist zum Beispiel bei der oben erwähnten, fertigungsbedingten wellenförmigen Oberflächenstruktur von Vorteil.

Die Schichtdicke der Zr-Beschichtung einer Implantatkomponente gemäß der vorliegenden Offenbarung liegt bevorzugt zwischen 3 µm und 6 µm, und besonders bevorzugt zwischen 3 µm und 5 µm.

Ziel ist es, die Dicke der Schicht so hoch zu wählen, dass eine den mechanischen Belastungen widerstehende Beschichtung erzielt wird. Gleichzeitig ist anzustreben, die Beschichtung nicht zu stark zu wählen, damit ein stabiler Reibschluss zwischen den Flächen des Verbindungsbereichs erreicht wird.

Die Herstellung einer Zr-Schicht mit einer großen Dicke ist mit höheren Kosten verbunden. Bei einer zu dünnen Schicht kann hingegen der angestrebte Effekt (Verringerung der Anfälligkeit für Spaltkorrosion und/oder Reibkorrosion) gegebenenfalls nicht dauerhaft und reproduzierbar erzielt werden. Die oben aufgeführten, (besonders) bevorzugten Schichtdicken sind mit vertretbar geringen Herstellungskosten verbunden und erlauben es gleichzeitig, die Anfälligkeit des Verbindungsabschnitts für Spaltkorrosion und/oder Reibkorrosion gegenüber bekannten Beschichtungen zu reduzieren. Die oben genannten Schichtdicken können somit auch als Lösung eines mehrdimensionalen Optimierungsproblems hinsichtlich Herstellungskosten und Eintritt des angestrebten Effekts angesehen werden.

Eine Implantatkomponente gemäß der vorliegenden Offenbarung kann einen Verbindungsabschnitt aufweisen, der mit einem weiblichen und/oder männlichen Verjüngung gebildet ist. Bevorzugt ist die weibliche und/oder männliche Verjüngung rotationssymmetrisch.

Eine Verjüngungsverbindung kann durch ihre verkeilenden Eigenschaften als selbststabilisierend angesehen werden. Zudem zeichnet sie sich durch verhältnismäßig geringe Fertigungskosten aus und erlaubt eine präzise Fertigung der Passflächen (beispielsweise mit einer Rundlauftoleranz unter 0,1 mm, unter 0,05 mm oder unter 0,01 mm). Als Folge der hohen Fertigungspräzision, die bei derartigen Geometrien erreicht werden kann, kann der Betrag des freien Volumens zwischen Passflächen auf ein Minimum reduziert werden. Dadurch werden die über die Verjüngungsverbindung übertragenen Kräfte gleichmäßig abgetragen. Spannungsspitzen werden verhindert.

Wenn die Verjüngungsverbindung rotationssymmetrisch ausgebildet ist, steht dem operierenden Chirurgen während des Einsetzens des Implantats ein weiterer Freiheitsgrad zum Ausrichten des Implantats zur Verfügung, nämlich ein Drehfreiheitsgrad um die Verjüngungsachse. Dieser Drehfreiheitsgrad erlaubt es bei bestimmten Implantaten, die Implantatgeometrie schnell und einfach an die Patientengeometrie anzupassen.

Alternativ zur Verjüngungsverbindung kann der Verbindungsabschnitt beispielswese als achsparalleler Zylinder und/oder als achsparallele Bohrung ausgebildet sein. Der achsparallele Zylinder und/oder die achsparallele Bohrung kann, wenn der Verbindungsabschnitt mit einer anderen Implantatkomponente verbunden ist, teil eines Passsystems sein, beispielsweise mit einer Presspassung oder einer Übergangspassung (z.B. H7p6 oder H7n6).

Der Verbindungsabschnitt kann ferner einen Anschlag mit einer Anschlagfläche aufweisen, wobei die Anschlagfläche beispielsweise im Wesentlichen senkrecht auf der Verjüngungs- bzw. Zylinderachse stehen kann oder einen Winkel mit dieser einschließen kann. Die Anschlagfläche ist bevorzugt im Wesentlichen rotationssymmetrisch bezüglich der Verjüngungs- bzw. Zylinderachse ausgebildet.

Eine Implantatkomponente gemäß der vorliegenden Offenbarung kann eine Metalllegierung, beispielsweise eine Titanbasislegierung oder eine Kobaltbasislegierung, aufweisen. Bevorzugt ist die Implantatkomponente im Wesentlichen aus einer Metalllegierung, beispielsweise einer Titanbasislegierung oder einer Kobaltbasislegierung ausgebildet. Mit anderen Worten ist die Zr-Beschichtung bevorzugt auf einen Verbindungsabschnitt einer im Wesentlichen aus einer der vorstehend genannten Legierungen gebildeten Implantatkomponente aufgebracht. Insbesondere kann eine offenbarungsgemäße Implantatkomponente eine CoCr-Legierung aufweisen, die beispielsweise ca. 62-66 Gew.-% Kobalt, ca. 27-31 Gew.-% Chrom und 4-5 Gew.-% Molybdän enthält. Eine derartige Legierung kann in geringen Mengen jedoch auch Kohlenstoff, Silizium, Mangan, Eisen und/oder weitere Begleitelemente enthalten (z. B. gemäß ISO 5832-4, ASTM F75). Weitere Beispiele von Metalllegierungen, die bei einer Implantatkomponente zum Einsatz kommen können, sind CoCr-Schmiedelegierungen (z. B. gemäß ISO 5832-12), Stahllegierungen (z. B. gemäß ISO 5832-1) oder Titanlegierungen (z. B. gemäß ISO 5832-3 oder ISO 5832-11).

Die Implantatkomponente kann jedoch auch andere Werkstoffe, beispielsweise eine Keramik oder eine Kunststoffsorte aufweisen, oder im Wesentlichen aus einem oder mehreren dieser Werkstoffe gebildet sein. Als Keramikwerkstoffe können beispielsweise Aluminium-, Titan-, Zirconium- und/oder Magnesiumbasierte Keramiken verwendet werden. Als Kunststoffsorten kommen beispielsweise UHMW-PE, PP, PEEK oder POM in Frage.

Die genannten metallischen Werkstoffe bieten beispielsweise eine hohe mechanische Festigkeit in Verbindung mit ausgezeichneten Eigenschaften bezüglich Biokompatibilität. Zudem sind insbesondere Titanlegierungen für ein ausgezeichnetes Einwachsverhalten von Knochengewebe bekannt. Die genannten Keramikwerkstoffe können Vorteile bezüglich der Dauerfestigkeit aufweisen. Die obigen Kunststoffsorten können vorteilhafte Gleiteigenschaften, eine vorteilhafte Schlagzähigkeit, eine vorteilhaft hohe Duktilität und/oder ausgezeichnete gewichtsbezogene Festigkeitseigenschaften aufweisen.

Eine Implantatkomponente gemäß der vorliegenden Offenbarung kann beispielsweise ein Prothesenschaft, ein Zwischenstück oder eine Gelenkkomponente, insbesondere ein Gelenkkopf, sein. Die Implantatkomponente kann beispielsweise dazu ausgebildet sein, als Bestandteil einer Hüftgelenkersatzprothese, einer Kniegelenkersatzprothese, einer Ellenbogengelenkersatzprothese, einer Schultergelenkersatzprothese oder einer Fuß-, Hand,- oder Fingergelenkersatzprothese eingesetzt zu werden.

Bei einer offenbarungsgemäßen Implantatkomponente kann die Zr-Beschichtung beispielsweise einen Zr-Anteil von zumindest 90 At.-%, aufweisen. Bevorzugt weist die Zr-Beschichtung einen Zr-Anteil von zumindest 94 At.-% und besonders bevorzugt von zumindest 99,5 At-% auf. Die Angaben sind als Stoffmengenanteile zu verstehen. Bei einer hohen Reinheit der Beschichtung, beispielsweise bei den oben genannten Zr-Anteilen, kann eine Verringerung der Anfälligkeit für Spaltkorrosion und/oder Reibkorrosion besonders gut ausgeprägt sein.

Bei einer offenbarungsgemäßen Implantatkomponente kann die Beschichtung durch ein physikalisches Gasphasenabscheidungsverfahren oder durch ein Galvanisierungsverfahren aufgebracht sein. Ein Galvanisierungsverfahren kann gegenüber anderen Verfahren Kostenvorteile aufweisen. Ein Gasphasenabscheidungsverfahren (z.B. PVD-Verfahren) zeichnet sich durch eine hohe Genauigkeit bei der Einhaltung der angestrebten Schichtdicke aus. Beispielsweise können mit einem PVD-Verfahren Abweichungen von nicht mehr als ±20% (bezogen auf eine Sollschichtdicke) erreicht werden. Insbesondere können mit Gasphasenabscheidungsverfahren auch bei komplexen Geometrien sehr gleichförmige Schichtdicken erzeugt werden. Eine Beschichtung, die mit einem Gasphasenabscheidungsverfahren aufgebracht ist, haftet zudem wesentlich besser an der Oberfläche des beschichteten Werkstücks als eine Beschichtung, die mit einem anderen Verfahren aufgebracht worden ist.

Gemäß der vorliegenden Offenbarung wird ferner eine modulare Endoprothese bereitgestellt, die zumindest eine, bevorzugt jedoch genau eine der oben beschriebenen offenbarungsgemäßen Implantatkomponenten aufweist. Bevorzugt weist die offenbarungsgemäße modulare Endoprothese ferner eine zweite Implantatkomponente mit einem Verbindungsabschnittgegenstück auf, wobei das Verbindungsabschnittgegenstück dazu ausgebildet ist, mit dem Verbindungsabschnitt der offenbarungsgemäßen Implantatkomponente in Eingriff zu gehen. In anderen Worten sind der Verbindungsabschnitt und das Verbindungsabschnittgegenstück komplementär ausgeführt. Das Verbindungsabschnittgegenstück weist womöglich oder weist eine Zr-Beschichtung auf.

Es hat sich gezeigt, dass eine Verringerung der Anfälligkeit für Spaltkorrosion und/oder Reibkorrosion bereits eintritt, wenn der Verbindungsabschnitt einer ersten Implantatkomponente einer modularen Endoprothese eine offenbarungsgemäße Zr-Beschichtung aufweist, während ein mit dem Verbindungsabschnitt verbundenes Verbindungsabschnittgegenstück einer zweiten Implantatkomponente womöglich keine Zr-Beschichtung aufweist. Jedoch können bei einer offenbarungsgemäßen modularen Endoprothese auch sowohl der Verbindungsabschnitt, als auch das mit dem Verbindungsabschnitt verbundene Verbindungsabschnittgegenstück einer zweiten Implantatkomponente eine Zr-Beschichtung aufweisen.

Bei einer offenbarungsgemäßen modularen Endoprothese kann die erste Implantatkomponente beispielsweise ein Prothesenschaft sein, bei dem der Verbindungsabschnitt eine männliche Verjüngung ist, und die zweite Implantatkomponente kann beispielsweise eine Gelenkkomponente, insbesondere ein Gelenkkopf sein, bei dem das Verbindungsabschnittgegenstück als weibliche Verjüngung ausgeführt ist.

Zudem wird auch die Verwendung einer Zr-Beschichtung zur Vorbeugung gegen Korrosion an einem Verbindungsabschnitt einer Implantatkomponente offenbart, wobei die Beschichtung eine Dicke von 1-20 µm, bevorzugt 1-6 µm und besonders bevorzugt 3-5 µm aufweist, und wobei die Implantatkomponente bevorzugt eine der vorstehend beschriebenen offenbarungsgemäßen Implantatkomponenten ist. Diese Verwendung weist die Gleichen oder vergleichbare Vorteile bzw. Effekte auf wie eine offenbarungsgemäße Implantatkomponente bzw. eine offenbarungsgemäße modulare Endoprothese.

Ferner wird auch die Verwendung einer Implantatkomponente oder einer Endoprothese als Hüftgelenkersatz bei einem Patienten mit Metallallergie offenbart. In diesem Zusammenhang wird darauf hingewiesen, dass verschiedene Patienten verschiedenartige Reaktionen auf das Auftreten von Spaltkorrosion und/oder Reibkorrosion zeigen können. So ist es beispielsweise denkbar, dass ein erster Patient keine erkennbare Reaktion auf Stoffe zeigt, die aufgrund von Spaltkorrosion und/oder Reibkorrosion freigesetzt werden. Hingegen kann ein zweiter Patient allergische Reaktionen auf etwaige Stoffe zeigen, die aufgrund von Spaltkorrosion und/oder Reibkorrosion freigesetzt werden. Somit treten die Vorteile einer offenbarungsgemäßen Implantatkomponente bzw. einer offenbarungsgemäßen modularen Endoprothese insbesondere bei Patienten zu Tage, bei denen die vorstehen beschriebenen allergischen Reaktionen auftreten können.

### KURZE BESCHEIRUNG DER ZEICHNUNGEN

- Fig. 1: zeigt eine Ausführungsform einer modularen Endoprothese gemäß der vorliegenden Offenbarung im zerlegten Zustand;
- Fig. 2: zeigt die Ausführungsform der modularen Endoprothese aus Fig. 1 in einem verbundenen Zustand.
- Fig. 3a: zeigt den Verlauf der Stromstärke, die bei zyklischer Belastung einer Endoprothese mit einer bekannten Implantatkomponente gemessen wurde;
- Fig. 3b: zeigt den Verlauf der Stromstärke, die bei zyklischer Belastung einer Endoprothese mit einer Implantatkomponente gemäß der vorliegenden Offenbarung gemessen wurde.

### AUSFÜHRLICHE BESCHREIBUNG BEVORZUGTER AUSFÜHRUNGSFORMEN

Die nachfolgend beschriebenen bevorzugten Ausführungsformen stellen lediglich Beispiele dar und sind nicht als beschränkend anzusehen. Gleiche Bezugszeichen, die in verschiedenen Figuren aufgeführt sind, benennen identische, einander entsprechende, oder funktionell ähnliche Elemente.

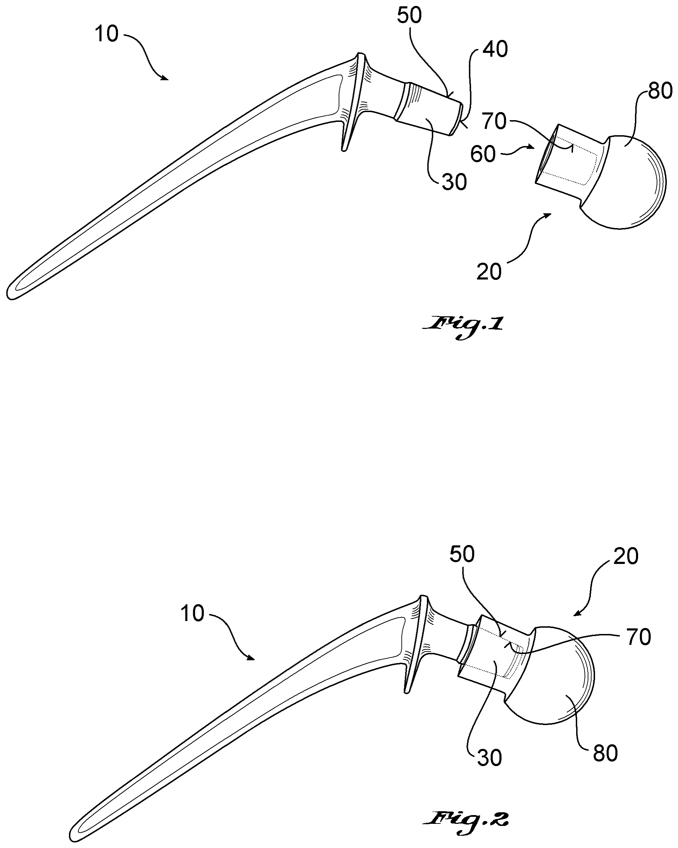

Fig. 1 zeigt eine Ausführungsform einer modularen Endoprothese gemäß der vorliegenden Offenbarung in einem nichtverbundenen Zustand. Die modulare Endoprothese weist als erste Implantatkomponente einen Prothesenschaft (10), und als zweite Implantatkomponente einen Gelenkkopf (20) auf. Der Prothesenschaft (10) weist einen Verbindungsabschnitt auf, der als männliche Verjüngung (30) ausgebildet ist. Die männliche Verjüngung (30) weist eine Stirnfläche (40) und eine äußere Umfangsfläche (50) auf. Die männliche Verjüngung (30) ist zumindest abschnittsweise mit einer Zr-Beschichtung beschichtet, wobei die Zr-Beschichtung eine Schichtdicke von 1-20 µm, bevorzugt 1-6 µm aufweist. Bevorzugt ist zumindest die gesamte äußere Umfangsfläche (50) der männlichen Verjüngung (30) mit einer Zr-Beschichtung beschichtet. Ferner kann zusätzlich die Stirnfläche (40) der männlichen Verjüngung (30) mit einer Zr-Beschichtung beschichtet sein. Der Übergang von der äußeren Umfangsfläche (50) der männlichen Verjüngung (30) hin zur Stirnfläche (40) der männlichen Verjüngung (30) kann beispielsweise als Fase oder Kantenverrundung ausgebildet sein. Auch dieser Übergang (d.h., die Fase bzw. Kantenverrundung) kann eine offenbarungsgemäße Zr-Beschichtung aufweisen.

Der Gelenkkopf (20) weist ein Verbindungsabschnittgegenstück auf, das als weibliche Verjüngung (60) ausgebildet ist. Die weibliche Verjüngung (60) weist eine innere Umfangsfläche (70) und eine Bodenfläche auf. Der Gelenkkopf (20) weist ferner eine Gelenkkugel (80) auf. Die Gelenkkugel ist bevorzugt als Kugelsegment mit einer im Wesentlichen sphärischen Oberfläche ausgebildet, die als Gelenkfläche dieser Gelenkkomponente dient. In den Figuren 1 und 2 ist die weibliche Verjüngung (60) in einem der Gelenkkugel im Wesentlichen gegenüberliegenden Anschlussbereich ausgebildet. Der Anschlussbereich mit der darin ausgebildeten weiblichen Verjüngung (60) steht von der Seite der Gelenkkugel hervor, welcher das Kugelsegment begrenzt. Die weibliche Verjüngung (60) kann sich dabei bis in den Bereich des Kugelsegments erstrecken. Ebenso ist es möglich, dass der Anschlussbereich nicht als Vorsprung, sondern als Fläche ausgebildet ist, die das Kugelsegment und damit die Gelenkfläche definiert. In diesem Fall ist die weibliche Verjüngung (60) innerhalb des Kugelsegments ausgebildet.
In der vorliegenden Ausführungsform weist weder die innere Umfangsfläche (70), noch die Bodenfläche der weiblichen Verjüngung (60) eine Zr-Beschichtung auf. In einer Abwandlung der oben beschriebenen Ausführungsform kann jedoch auch die weibliche Verjüngung (60) des Gelenkkopfs (20) eine Zr-Beschichtung aufweisen, während die männliche Verjüngung (30) des Prothesenschafts (10) keine Zr-Beschichtung aufweist. Gemäß einer weiteren Modifikation können sowohl die weibliche Verjüngung (60) des Gelenkkopfs (20) als auch die männliche Verjüngung (30) des Prothesenschafts (10) eine Zr-Beschichtung aufweisen. Bevorzugt ist zumindest die gesamte äußere Umfangsfläche (50) der männlichen Verjüngung (30) oder die gesamte innere Umfangsfläche (70) der weiblichen Verjüngung (60) mit einer Zr-Beschichtung beschichtet.

In einer nicht in den Figuren 1 und 2 dargestellten Variante der oben beschrieben Ausführungsform können die weibliche Verjüngung (60) und die männliche Verjüngung (30) auch vertauscht sein. So kann beispielsweise auch der Prothesenschaft (10) eine weiblichen Verjüngung aufweisen, und der Gelenkkopf (20) kann eine männlichen Verjüngung aufweisen.

In Fig. 2 ist die oben beschriebene Ausführungsform der offenbarungsgemäßen modularen Endoprothese in einem verbundenen Zustand dargestellt. Aus Fig. 2 geht hervor, dass die innere Umfangsfläche (70) der weiblichen Verjüngung (60) im verbundenen Zustand mit der äußeren Umfangsfläche (50) der männlichen Verjüngung (30) in Kontakt steht. Um eine Doppelpassung (die einem Verkeilungseffekt der männlichen Verjüngung (30) und der weiblichen Verjüngung (60) ggf. entgegenstehen könnte) zu vermeiden, ist es bevorzugt, dass die Geometrie der männlichen Verjüngung (30) und die Geometrie der weiblichen Verjüngung (60) derart aufeinander abgestimmt sind, dass die Stirnfläche (40) der männlichen Verjüngung (30) und die Bodenfläche der weiblichen Verjüngung (60) nicht in Kontakt stehen.

Wenn die Stirnfläche (40) der männlichen Verjüngung (30) und die Bodenfläche der weiblichen Verjüngung (60) (und/oder etwaige Fasen, Radien oder Übergangsbereiche) nicht miteinander in Kontakt stehen, ist davon auszugehen, dass an diesen Flächen keine Spaltkorrosion und/oder Reibkorrosion auftritt. Somit ist eine Zr-Beschichtung an diesen Flächen nicht notwendig. Dennoch kann an diesen Flächen eine Zr-Beschichtung vorgesehen sein. Insbesondere kann es aus anderen Gründen vorteilhaft sein, an der Stirnfläche (40) der männlichen Verjüngung (30), an der Bodenfläche der weiblichen Verjüngung (60) und/oder an etwaigen Fasen, Radien oder Übergangsbereichen eine Zr-Beschichtung vorzusehen. Wenn auch in diesen Bereichen eine Zr-Beschichtung vorgesehen ist, kann dort beispielsweise ein Übergang von einer unbeschichteten Fläche zu einer beschichteten Fläche verhindert werden, wodurch wiederum das Risiko reduziert wird, dass Teile der beschichteten Fläche abplatzen (z.B. infolge einer Kerbwirkung und/oder infolge von Spannungsspitzen). Darüber hinaus entfällt (wenn auch die Stirnfläche (40) der männlichen Verjüngung (30), die Bodenfläche der weiblichen Verjüngung (60) und/oder etwaige Fasen, Radien oder Übergangsbereiche mitbeschichtet werden) die Notwendigkeit, diese Flächen während des Beschichtungsvorgangs zu maskieren. Dadurch können wiederum Kostenvorteile erzielt werden.

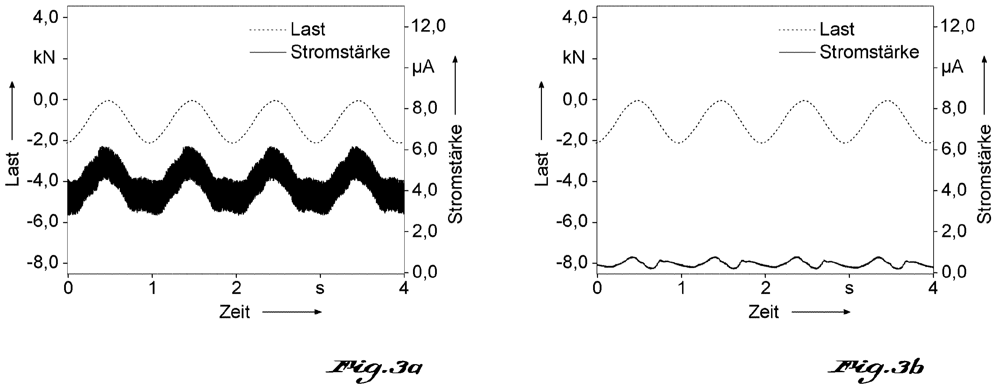

Die Figuren 3a und 3b veranschaulichen (auszugsweise) Messwerte einer experimentellen Untersuchung, bei der eine bekannte Implantatkomponente (Fig. 3a) und eine Implantatkomponente mit einer offenbarungsgemäßen Zr-Beschichtung (Fig. 3b) in Bezug auf das Auftreten von Spaltkorrosion und/oder Reibkorrosion untersucht wurden. Qualitative bzw. vergleichende Informationen über die Anfälligkeit eines Verbindungsabschnitts für Spaltkorrosion und/oder Reibkorrosion können als experimentelle Untersuchung beispielsweise mit einem Messverfahren nach ASTM F1875 - 98 (erneut zugelassen 2014) erlangt werden. Bei diesem Verfahren werden femorale Schaft- und Kopfkomponenten in ein Medium, beispielsweise eine Kochsalzlösung, eingebracht und mit einer zyklischen Last beaufschlagt. Die Schaft- und Kopfkomponenten sind mittels eines Verbindungsabschnitts verbunden. Ebenfalls in das Medium eingebracht werden Referenzelektroden, deren Beschichtungsmaterial mit dem Beschichtungsmaterial der zu prüfenden Schaft- und Kopfkomponenten übereinstimmt. Auch die Oberflächengröße der Referenzelektroden und die Oberflächengröße der (in das Medium eingebrachten Abschnitte der) Schaft- und Kopfkomponenten entsprechen einander.

Die Schaft- und Kopfkomponenten sowie die Referenzelektroden sind mit einem Strommessgerät verbunden, das es erlaubt, Ströme zu messen, die (über das Medium) zwischen den Schaft- und Kopfkomponenten einerseits und den Referenzelektroden andererseits fließen. Da die Oberfläche und das Beschichtungsmaterial der Schaft- und Kopfkomponenten und der Referenzelektroden identisch sind, sind diese Ströme jedoch nicht auf einen Potenzialunterschied zwischen den Schaft- und Kopfkomponenten und den Referenzelektroden zurückzuführen (Galvanische Zelle/Batterieeffekt). Vielmehr resultiert der messbare Stromfluss zwischen den Schaft- und Kopfkomponenten einerseits und den Referenzelektroden andererseits daraus, dass infolge der oben genannten zyklischen Krafteinwirkung Teile der Passivschicht (bzw. der Passivschichten) an der Oberfläche (bzw. den Oberflächen) des Verbindungsabschnitts abgerieben werden und sich neu bilden.

Aus den Messwerten bezüglich des Stromflusses zwischen den Schaft- und Kopfkomponenten und den Referenzelektroden können die im zeitlichen Verlauf gemittelte Stromstärke Iₘ, und die mittlere dynamische Stromstärke I_{d} bestimmt werden. Bei der mittleren dynamischen Stromstärke I_{d} handelt es sich um die Differenz aus der in einem bestimmten Zeitintervall gemessenen maximalen Stromstärke Iₘₐₓ und der minimalen Stromstärke Iₘᵢₙ (d.h., im Zeitintervall Δt1 gilt: I_{d,Δt1} = I_{max,Δt1} - I_{min,Δt1}).

Wenn bei einer ersten Kombination aus Schaft- und Kopfkomponenten ein geringerer Wert Iₘ gemessen wird als bei einer zweiten Kombination aus Schaft- und Kopfkomponenten, gilt dies als indirekter Nachweis dafür, dass die erste Kombination aus Schaft- und Kopfkomponenten weniger anfällig für Spaltkorrosion und/oder Reibkorrosion ist, als die zweite Kombination aus Schaft- und Kopfkomponenten.

Insbesondere gilt, wenn bei einer ersten Kombination aus Schaft- und Kopfkomponenten ein geringerer Wert Iₘ und ein geringerer Wert I_{d} gemessen wird als bei einer zweiten Kombination aus Schaft- und Kopfkomponenten, dies als indirekter Nachweis dafür, dass die erste Kombination aus Schaft- und Kopfkomponenten signifikant weniger anfällig für Spaltkorrosion und/oder Reibkorrosion ist als die zweite Kombination aus Schaft- und Kopfkomponenten.

Die bekannte Implantatkomponente der Fig. 3a und die Implantatkomponente mit offenbarungsgemäßer Zr-Beschichtung der Fig. 3b waren jeweils mit einem Verbindungsabschnitt mit einer weiteren Implantatkomponente verbunden, wodurch jeweils eine Endoprothese gebildet wurde. Die Geometrie der Endoprothese mit der bekannten Implantatkomponente, und die Geometrie der Endoprothese mit der Implantatkomponente mit offenbarungsgemäßer Zr-Beschichtung waren identisch. Ferner wurden bei beiden Endoprothesen identische Versuchsparameter gewählt. Wie aus den Figuren 3a und 3b hervorgeht, wurden die Endoprothesen mit einer zyklischen Last beaufschlagt, deren Frequenz 1 Hz betrug. Der Betrag der Last verlief periodisch zwischen 0,04 kN und 2,04 kN. In den Diagrammen in Fig. 3a und Fig. 3b ist die Last (in Kilonewton) auf der jeweils linken vertikalen Achse aufgetragen, wobei das negative Vorzeichen der Last auf deren Orientierung zurückzuführen ist (Drucklast). Auf der jeweils horizontalen Achse der Diagramme ist die Zeit in Sekunden aufgetragen. Auf der jeweils rechten vertikalen Achse ist die Stromstärke (in Microampere) aufgetragen, die während der zyklischen Belastung der Endoprothesen zwischen den Endoprothesen und den Referenzelektroden gemessen wurde.

Wie anhand von Fig. 3a nachzuvollziehen ist, wurden in einem bestimmten Zeitintervall bei der Endoprothese mit der bekannten Implantatkomponente eine im zeitlichen Verlauf gemittelte Stromstärke Iₘ - 4,49 µA, und eine mittlere dynamische Stromstärke I_{d} = 3,61 µmA gemessen. Wie anhand von Fig. 3b zu erkennen ist, wurden in einem bestimmten Zeitintervall bei der Endoprothese mit der Implantatkomponente mit der offenbarungsgemäßen Zr-Beschichtung eine im zeitlichen Verlauf gemittelte Stromstärke Iₘ = 0,49 µA, und eine mittlere dynamische Stromstärke I_{d} = 0,68 µmA gemessen. Ein Vergleich der Versuchsreihen lässt den Schluss zu, dass die Endoprothese mit der Implantatkomponente mit der offenbarungsgemäßen Zr-Beschichtung weniger anfällig für Spaltkorrosion und/oder Reibkorrosion ist als die Endoprothese mit der bekannten Implantatkomponente.

## Patentansprüche

1. Implantatkomponente (10, 20), mit zumindest einem Verbindungsabschnitt (30, 60), wobei der Verbindungsabschnitt (30, 60) eine röhrenförmige Oberfläche (50, 70) umfasst, wobei zumindest eine der röhrenförmigen Oberflächen (50, 70) zumindestends abschnittsweise mit einer Zr-Beschichtung beschichtet sind und die Beschichtung eine Dicke von 1-20 µm, bevorzugt 1-6 µm aufweist,
**dadurch gekennzeichnet, dass**
die Zr-Beschichtung einen Zr-Anteil von zumindest 90 At.-% aufweist, und dass die Zr-Beschichtung zumindest eine von elementarem Zirconium und Zirconium-basierte Oxide umfasst.

2. Implantatkomponente (10) nach Anspruch 1, bei der die Beschichtung eine Dicke von 3-6 µm, bevorzugt von 3-5 µm aufweist.

3. Implantatkomponente (10) nach einem der vorstehenden Ansprüche, bei welcher der Verbindungsabschnitt (30, 60) eine weibliche und/oder männliche Verjüngung aufweist.

4. Implantatkomponente (10) nach einem der vorstehenden Ansprüche, bei welcher der Verbindungsabschnitt (30) rotationssymmetrisch ist.

5. Implantatkomponente (10) nach einem der vorstehenden Ansprüche, wobei die Implantatkomponente (10) eine Metalllegierung, bevorzugt eine Titanbasislegierung oder eine Kobaltbasislegierung, aufweist.

6. Implantatkomponente (10) nach einem der vorstehenden Ansprüche, wobei die Implantatkomponente (10) eine CoCr-Legierung aufweist.

7. Implantatkomponente (10) nach einem der vorstehenden Ansprüche, wobei die Implantatkomponente (10) ein Prothesenschaft (10) ist.

8. Implantatkomponente (10) nach einem der vorstehenden Ansprüche, bei der die Zr-Beschichtung einen Zr-Anteil von zumindest 97 At.-%, bevorzugt zumindest 99,5 At.-% aufweist.

9. Implantatkomponente (10) nach einem der vorstehenden Ansprüche, bei der die Beschichtung durch ein physikalisches Gasphasenabscheidungsverfahren oder durch ein Galvanisierungsverfahren aufgebracht ist.

10. Implantatkomponente (10) nach einem der vorstehenden Ansprüche, bei der die Zr-Beschichtung Zirkon (Zr[SiO₄]), auf Zirconium basierende Mineralien, und Zirconium-keramiken ausschließt.

11. Modulare Endoprothese mit zumindest einer, bevorzugt jedoch genau einer ersten Implantatkomponente (10) nach einem der vorstehenden Ansprüche.

12. Modulare Endoprothese nach Anspruch 11, die ferner eine zweite Implantatkomponente (20) mit einem Verbindungsabschnittgegenstück (60) aufweist, wobei das Verbindungsabschnittgegenstück (60) dazu ausgebildet ist, mit dem Verbindungsabschnitt (30) der ersten Implantatkomponente (10) in Eingriff zu gehen, wobei das Verbindungsabschnittgegenstück (60) vorzugsweise keine Zr-Beschichtung aufweist.

13. Modulare Endoprothese nach Anspruch 12, bei der die erste Implantatkomponente ein Prothesenschaft (10) mit einem als männlicher Verjüngung ausgebildeten Verbindungsabschnitt (30) ist, und bei der die zweite Implantatkomponente ein Gelenkkopf (20) mit einem als weiblicher Verjüngung ausgebildeten Verbindungsabschnittgegenstück (60) ausgebildet ist.

14. Modulare Endoprothese nach Anspruch 10, die ferner eine zweite Implantatkomponente (20) mit einem Verbindungsabschnittgegenstück (60) aufweist, wobei das Verbindungsabschnittgegenstück (60) dazu ausgebildet ist, mit dem Verbindungsabschnitt (30) der ersten Implantatkomponente (10) in Eingriff zu gehen, wobei das Verbindungsabschnittgegenstück (60) zumindest teilweise mit einer Zr-Beschichtung beschichtet ist welche ein Zr Anteil von mindestens 90 At.-% hat und die Zr-Beschichtung zumindest eine von elementarem Zirconium und Zirconium-basierte Oxide umfasst.

15. Verwendung einer Zr-Beschichtung zur Vorbeugung gegen Korrosion an einem Verbindungsabschnitt (30) einer Implantatkomponente (10), wobei die Beschichtung eine Dicke von 1-20 µm, bevorzugt 1-6 µm und besonders bevorzugt 3-5 µm aufweist, und wobei die Implantatkomponente (10) eine Implantatkomponente (10) nach einem der Ansprüche 1 bis 9 ist.
